# EUROPEAN PATENT APPLICATION

(11) **EP 2 543 394 A1**
(43) Date of publication of application: **09.01.2013**
(21) Application number: 12187110.7
(22) Date of filing: 18.06.2009
(51) Int. Cl.: A61L 24/00, A61L 24/10

(54) **A method for enzymatic cross-linking of a protein**

(30) Priority: 18.06.2008 US 129321 P
(62) Divisional of application: 09766288.6
(71) Applicant: Lifebond Ltd, 38900 Caesarea (IL)
(72) Inventor: Attar, Ishay, 30815 Kibutz Nachsholim (IL); Preiss-Bloom, Orahn, 30900 Zichron Yakov (IL); Tomer, Guy, 71700 Modiin (IL)
(74) Representative: Virdee-Crofts, Kulwinder Kaur

(57) **Abstract**

Gel composition comprising reduced and denatured albumin and an enzymatic cross-linker for use as a medical sealant in applications such as reinforcement of surgical repair lines, provision of fluid -stasis, prevention of lymphorrea, or sealing of an attachement between a tissue and a material.

## Description

### FIELD OF THE INVENTION

The present invention relates to protein cross-linking, and more specifically to a method of improving enzymatic cross-linking of globular proteins using a structural modifier of the protein, and gels produced by this method.

### BACKGROUND OF THE INVENTION

Proteins which are able to undergo rapid cross-linking *in situ* are successfully utilized in a number of medical applications, such as in sealants and hemostats, in drug delivery, and in tissue engineering.

Nonetheless, existing products based on cross-linkable proteins have major flaws. For example, BioGlue® (CryoLife Inc., USA), a surgical adhesive composed of purified bovine albumin cross-linked by gluteraldhyde, is highly toxic and thus approved only for certain limited surgical applications, and not for general surgery.

Other synthetic and fibrin-based sealants are commonly used in the operating room, despite their low adhesive strength, which leads to significantly low efficacy.

The use of enzymes for cross-linking of certain proteins has previously been suggested, such as in PCT Application No. PCT/US07/25726, filed on December 17 2007 to some of the current inventors. A disadvantage of protein cross-linking by an enzymatic method is that such cross-linking reactions can only successfully be performed with proteins that contain reactive groups sufficiently accessible to the cross-linking enzymes. Examples of such proteins include casein, gelatin and wheat gluten.

Sealants comprising enzymatically cross-linked gelatin are known. Non-gelatin sealants have the advantage that, unlike gelatin, they can form solutions that remain in liquid phase at room temperature without the need for additives and thus will not necessitate any heating before use, such as in the operating room.

Furthermore, non-gelatin sealants can be much less viscous. The high viscosity of gelatin is also problematic for application via a surgical applicator nozzle. Spraying gelatin, even in solution, is very difficult, in contrast to spraying less viscous protein solutions, which is far easier.

Globular proteins, on the other hand, have a structure that makes their reactive groups insufficiently accessible for enzymatic protein cross-linking. Examples of globular proteins include β-lactoglobulin, α-lactalbumin, serum albumin and ovalbumin, recombinant human albumin and more, as described in PCT application PCT/NL05/000582, which describes the need for methods for improving accessibility of globular proteins for enzymatic cross-linking for industrial food manufacturing applications.

Several groups have improved the accessibility of globular proteins for enzymatic cross-linking for industrial food manufacturing applications. De Jong at al. (J. Agric. Food. Chem, 2001(49), 3389-3393) used microbial transglutaminase (mTG) to polymerize a 0.5% bovine serum albumin (BSA) solution that had been treated with dithiothreitol (DTT), a strong reducing agent. Nonaka et al (Agricultural and Biological Chemistry, 1989 (53, 10), 2619-2623) used mTG to polymerize a 1% BSA solution that has been treated with DTT. Both groups demonstrated mTG-catalyzed polymerization using SDS-PAGE but did not succeed in forming a mTG-crosslinked gel.

Kang et al (J Food Sci, 2003 (68, 7), 2215-2220) used BSA as an emulsifier in an oil in water emulsion, where the 5% w/v BSA emulsion was treated with mTG in the presence of 2-mercaptoethanol (2-ME) to form a BSA-stabilized emulsion gel. In that case, formation of the emulsion gels depended on the presence of 2-ME and enzymatic crosslinking was not essential for the gel formation process.

Gan et al (Food Hydrocolloids, 2009 (23), 1398-1405) used mTG alone or in combination with ribose followed by heat treatment (90° C for 3 hours) to form BSA gels from solutions of 10% BSA. Gelation in that case was induced by the denaturative heat treatment rather than mTG crosslinking. This is a well known technique in the art as many globular food proteins, such as albumin, can be gelled (coagulated) by heat treatment (Tobitani et al, Macromolecules, 1997 (30, 17), 4845-4854.

Other denaturative methods of globular protein gelation have been described. Thiol-dependent gelation process involves denaturation by the reduction of intramolecular disulfide bonds and subsequent protein aggregation due to formation of new intermolecular hydrogen bonds or new intermolecular disulfide bonds as a result of thiol-disulfide exchange. BSA has 17 pairs of disulfide bonds and therefore is very responsive to thiol reducing agents. This has been described by Hirose at al. (J Food Sci, (55, 4), 915 - 917). Denaturative agents have been described for use in causing gelation of whey proteins including BSA. Xiong et al. used urea for this purpose (J. Agric Food Chem, 1990 (38, 10), 1887-1891).

Thus, denaturative treatment can independently induce non-enzymatic gelation of the above globular proteins, such that even in cases in which enzymes were used to apparently induce cross-linking, in fact cross-linking was induced through non-enzymatic mechanisms such as those described above.

Among treatments that are used to denature globular proteins are: incubation with reducing agents, incubation with denaturing or chaotropic agents, and heating. Denaturation by these methods can result in protein aggregation, completely independent of an enzymatic crosslinker, as a result of intermolecular bonds formed between protein regions. Such aggregation can be in the form of precipitation or gelation. With increasing concentrations of globular protein, more extensive denaturation is required. Increasing the intensity of the denaturative treatment increases the resultant gelation or precipitation of the protein independently of any enzymatic cross-linking.

### SUMMARY OF THE INVENTION

There is a need for, and it would be useful to have, a method for enzymatically cross-linking globular proteins, which is devoid of at least some of the limitations of the prior art.

The present invention overcomes these drawbacks of the background art by providing, in some embodiments of the present invention, a method for cross-linking globular proteins without the use of a denaturing pre-treatment that results in aggregation or gelation of the globular protein solution, such that cross-linking effectively occurs through the activity of the enzyme. By "effectively occurs" it is mean that crosslinking which leads to formation of a solid gel occurs due to activity of the enzyme, even if a slight amount of cross-linking occurs due to application of a denaturing treatment or pre-treatment.

Such a method is useful for a variety of medical applications including but not limited to the use as a sealant or glue for a biological system, for example to induce hemostasis and/or prevent leakage of any other fluid from a biological tube or tissue, such as lymph, bile, urinary tract or gastrointestinal tract for example. The cross-linkable albumin and cross-linker may optionally and preferably be applied together with a bioabsorbable or non-bioabsorbable backing or bandage. In another optional embodiment, the components are absorbed, adsorbed or otherwise adhered to or combined with the backing or bandage for example.

In other embodiments, the present invention provides a method of enzymatically cross-linking globular proteins, by altering the structure of the protein to improve the accessibility of the protein to the cross-linking enzyme.

According to some embodiments of the present invention, there is provided a method for cross-linking a globular protein, the method comprising adding to a solution of the globular protein a cross-linking enzyme and a structural modifier of the globular protein.

"Globular proteins" is used herein in its art-recognized meaning and includes proteins that have a globular domain. Preferably, however, aspects of the present invention relate to proteins that are strictly globular, which for example in this context may optionally relate to those proteins that cannot be cross-linked without the use of structure-modifying agents.

According to some embodiments of the present invention, there is provided a method for cross-linking a globular protein, the method comprising adding to a solution of the globular protein a cross-linking enzyme and a structural modifier of the globular protein.

According to some embodiments of the present invention, there is provided a method for preparing a medical gel, the method comprising adding to a solution of a cross-linkable globular protein a cross-linking enzyme and a structural modifier of the globular protein.

Optionally and preferably, the globular protein is modified in a manner that at least partially disrupts its tertiary structure (i.e. at least partially denatures it) while maintaining the protein in a soluble state such that the protein solution remains in liquid form.

According to a preferred embodiment, the globular protein is denatured by heat treatment sufficient to disrupt tertiary and quaternary structures. In order for the denaturation to be effective, the globular protein should be heated at a temperature that is high enough to denature the protein and cause aggregation. However, the temperature range and heating time should be sufficiently low to ensure that the protein aggregation is reversible. The heating temperature is preferably from about 50°C to about 80°C, while the heating time is optionally from about 10 minutes to about 60 minutes, depending upon such factors as the nature of the protein itself, the nature of the composition containing the protein and its application, and the temperature selected.

According to a preferred embodiment, the heat-induced aggregation can be reversed by the addition of a suitable agent, including but not limited to a denaturing agent, a chaotropic agent, a disulfide bond reducing agent or some combination of these.

According to another embodiment, the protein is denatured by a chaotropic agent or denaturing agent. A chaotropic agent, also known as chaotropic reagent and chaotrope, is any substance which disrupts the three dimensional structure in macromolecules including but not limited to proteins, DNA, or RNA.

According to a preferred embodiment the chaotropic agent is selected from the group consisting of urea, guanidinium chloride, and lithium perchlorate.

According to a preferred embodiment, the chaotropic agent is urea.

According to a preferred embodiment, sufficient urea is added to break intramolecular hydrogen bonds to a sufficient degree to facilitate the formation of intermolecular disulfide bonds as a result of thiol-disulfide exchange to result in aggregation, in the form of either precipitation or gelation.

Preferably, the concentration of urea in the protein solution is in the range of from about 3M to about 7M, although again this concentration may optionally be adjusted according to the nature of the protein, the nature of the solution and its application, and the concentration of protein thereof. More preferably, the concentration is from 4M to 6M.

According to a preferred embodiment, precipitation or gelation resulting from thiol-disulfide exchange is prevented after globular protein solution is treated with a disulfide bond reducing agent under conditions that would normally result in precipitation or gelation. For example, the globular protein is optionally denatured by a disulfide bond reducing agent, which prevents thiol-disulfide exchange. A disulfide bond reducing agent is any substance that is able to reduce a disulfide bond (R-S-S-R) to 2 thiol groups (R-S-H).

According to a preferred embodiment, the disulfide bond reducing agent is a thiol containing reducing agent, including but not limited to 2-mercaptoethanol, DTT, cystein, glutathione, or some combination of these agents.

According to a preferred embodiment the disulfide bond reducing agent is selected from the group consisting of phosphine-containing agents, including but not limited to tris(2-carboxyethyl) phosphine (TCEP).

According to a more preferred embodiment, the reducing agent is TCEP.

Preferably, the concentration of TCEP is in the range of about 1mM to about 500mM, although again this concentration may optionally be adjusted according to the nature of the protein, the nature of the solution and its application, and the concentration of protein thereof. More preferably, the concentration of TCEP is from about 10mM to about 100mM.

In another embodiment, the reducing agent is added at a concentration that would normally result in thiol-induced gelation of the globular protein.

According to a preferred embodiment of the current invention, more than one denaturing method or agent is used either simultaneously or in series.

In a preferred embodiment, one denaturing method or agent disrupts hydrogen bonds while a second method or agent disrupts disulfide bonds.

According to a preferred embodiment a soluble denatured protein can be obtained by combining at least one chaotropic agent and at least one disulfide bond reducing agent with or without heat treatment. The heat treatment can be done prior to addition of chaotropic agent or disulfide bond reducing agent or both or concomitantly in their presence.

According to a preferred embodiment, the concentration of the reducing agent is reduced or the reducing agent is entirely removed prior to addition of crosslinking enzyme. This is desirable because reducing agents can adversely affect the crosslinking activity of enzymes (see example 4). Surprisingly, it was found that removing the reducing agent does not reverse its effect of disrupting disulfide bonding.

The removal can be done by methods known to those skilled in the art and include, but are not limited to, dialysis, ultrafiltration, size exclusion chromatography, and ammonium sulfate precipitation.

According to a preferred embodiment of the present invention salt may be added to denaturing mixtures of protein that contain chaotropic agents and reducing agents in order to prevent precipitation or gelation of protein that is triggered by heat treatment of the the mixture or prevent precipitation or gelation of protein that is triggered without prior heat treatment. The salt may be belong to a group that consists of NaCl, KCl,LiCl, MgCl2 or any other suitable salt. Preferably, the salt is NaCl and its concentration is in the range of 0.1M to 1M, although again this concentration may optionally be adjusted according to the nature of the protein, the nature of the solution and its application, and the concentration of protein thereof.. More preferably, the concentration of NaCl is from 0.25 to 0.5M.

According to some embodiments of the present invention, there is provided a gel composition comprising albumin and an enzymatic cross-linker.

According to some embodiments of the present invention, there is provided a gel composition, comprising a cross-linkable globular protein, a cross-linking enzyme and one or more structural modifiers of the globular protein. Optionally, one or more structural modifiers comprise denaturing agents. Optionally, the denaturing agents are present in an amount sufficient to at least disrupt tertiary structure of the globular protein. Preferably, the denaturing agents are present in an amount sufficient to disrupt tertiary and quaternary structures of the globular protein.

Optionally the denaturing agents are selected from the group consisting of a chaotropic agent, a disulfide bond reducing agent or some combination of these. Also optionally, the chaotropic agent is selected from the group consisting of urea, guanidinium chloride, and lithium perchlorate.

Optionally the disulfide bond reducing agent comprises one or more of a thiol containing reducing agent, including but not limited to 2-mercaptoethanol, dithiothreitol (DTT), cystein, glutathione, a phosphine-containing agent or a combination thereof.

Optionally the phosphine containing agent comprises tris(2-carboxyethyl) phosphine (TCEP).

Optionally the reducing agent is selected from the group consisting of hexafluoroisopropanol (HFIP), dimethyl sulfoxide (DMSO), sodium dodecyl sulfate (SDS), hydroquinone, 2-mercaptoethylamine, and N-ethylmaleimide. Optionally the ratio of urea to protein is in the range of 10-150 mmols urea /gram protein. Preferably the ratio is in the range of 25-120 mmols urea/ gram protein.

Optionally the ratio of TCEP to protein is 0.1-1.5 mmols TCEP/ gram protein. Preferably, the ratio is in the range of 0.25-1.25 mmols TCEP/gram protein. More preferably, the ratio is in the range of 10-30 micromoles 2-mercaptoethanol / gram albumin.

Optionally any of the compositions described herein further comprise a salt. Preferably, the salt comprises sodium chloride. Optionally, the denaturing agent comprises a combination of urea and TCEP. Preferably, a molar ratio of urea to TCEP is in the range of 10 to 1000. More preferably, the molar ratio is in the range of 20-500.

Optionally the globular protein is selected from the group consisting of soy protein, conalbumin, bovine serum albumin (BSA), hemoglobin, ovalbumin, α-chymotrypsinogen A, α-chymotrypsin, trypsin, trypsinogen, β-lactoglobulin, myoglobin, α-lactalbumin, lysozyme, ribonuclease A, and cytochrome c. Preferably, the globular protein comprises albumin. Optionally, the globular protein is present in an amount from about 2% to about 25 % w/w of the composition. Also optionally, the globular protein is present in an amount from about 5% to about 20% w/w of the composition.

Optionally the enzymatic cross-linker comprises trans glutaminase. Preferably, the trans glutaminase is calcium independent. More preferably, the trans glutaminase is microbial transglutaminase.

Optionally a protein content of the trans glutaminase is present in an amount from about 0.0001% to about 2 % w/w of the composition. Preferably, the transglutaminase is present in an amount of from about 0.01% to about 1% w/w of the composition. More preferably, the trans glutaminase is present in an amount of from about 0.1% to about 1% w/w of the composition. Most preferably, the transglutaminase is present in an amount of from about 0.5% to about 1.5% w/w of the composition.

Optionally the concentration of transglutaminase is in the range of from about 5 to about 100 enzyme units (U/mL) of total composition. Optionally the concentration of transglutaminase is in the range of from about 15 to about 55 enzyme units (U/mL) of total composition. Optionally the concentration of transglutaminase is in the range of from about 25 to about 45 enzyme units (U/mL) of total composition.

Optionally a ratio of cross linking material: cross linkable protein solution is about 10:1 to 1:10 v/v.

According to some embodiments there is provided an enzyme crosslinked globular protein gel composition. Optionally the globular protein is selected from the group consisting of soy protein, conalbumin, bovine serum albumin (BSA), hemoglobin, ovalbumin, α-chymotrypsinogen A, α-chymotrypsin, trypsin, trypsinogen, β-lactoglabulin, myoglobin, α-lactalbumin, lysozyme, ribonuclease A, and cytochrome c. Preferably, the globular protein comprises albumin.

According to some embodiments there is provided a gel composition, comprising cross-linked albumin at a concentration of at least 3% wt/wt albumin of the weight of the composition, wherein the albumin is present in solution during cross-linking. Optionally the concentration does not exceed about 10%. The composition optionally further comprises a reducing agent and a chaotropic agent. Optionally the concentration is at least about 10%. The composition also optionally further comprises a chaotropic agent.

Optionally the chaotropic agent is selected from the group consisting of urea, guanidinium chloride, and lithium perchlorate.

Optionally the ratio of urea to protein is in the range 50-250 mmols urea /gram globular protein

According to some embodiments there is provided a medical sealant, comprising the composition as described herein.

According to some embodiments there is provided a method of preparation of any of the above compositions, comprising: treating albumin with a reducing agent as described herein; and combining with the reduced albumin, a chaotropic denaturing agent as described herein and trans glutaminase to form a combination.

According to some embodiments there is provided a method of preparation of any of the above compositions, comprising: heating albumin; and combining the heated albumin, one or more denaturing agents as described herein and transglutaminase to form a combination.

According to some embodiments there is provided a method of maintaining open disulfide bond albumin in solution, comprising treating albumin in solution with a reducing agent to form open disulfide bond albumin; and removing the reducing agent while maintaining the denatured albumin in solution. Optionally the method further comprises treating the open disulfide bond albumin with a chaotropic denaturing agent prior to removal of the reducing agent.

According to some embodiments there is provided a method of preparation of a tertiary structure denatured albumin solution comprising: combining albumin with one or more denaturing agents, wherein the concentration of albumin in the solution is at least about 5% w/w. Optionally, the albumin concentration in solution is less than about 15% w/w. Also optionally, the albumin concentration in solution is greater than about 10%. Optionally the denaturing agent comprises a reducing agent and a chaotropic agent. Optionally, a salt is additionally combined with the albumin and denaturing agent.

Optionally the reducing agent is removed from the tertiary structure denatured albumin solution and the tertiary structure remains denatured. Optionally, the reducing agent is removed using dialysis or ultrafiltration. Optionally the denaturing agent comprises a chaotropic agent. Optionally the albumin is heated.

Optionally a crosslinking enzyme is combined with the tertiary structure denatured albumin solution to form a gel.

As described herein any of the methods may optionally be used to produce a product, which may also optionally be used as a medical sealant or glue.

According to some embodiments there is provided use of cross-linked albumin as a medical sealant or glue. According to some embodiments there is provided use of albumin and a cross-linking enzyme as a medical sealant or glue.

According to some embodiments there is provided use of the medical sealant of in a medical application selected from the group consisting of reinforcement of surgical repair lines; provision of fluid-stasis; prevention of lymphorrhea; prevention of cerebro-spinal fluid (CSF) leakage; prevention of anastomotic dehiscence; and sealing of an attachment between a tissue and a material.

Optionally the medical sealant is provided in a form selected from the group consisting of a gel, a spray, a strip, a patch, and a bandage.

The composition as described herein may optionally be used for preparation of a tissue engineering scaffold. According to some embodiments there is provided use of enzyme cross-linked albumin as a tissue engineering scaffold.

According to some embodiments there is provided use of albumin and a cross-linking enzyme as a tissue engineering scaffold.

According to some embodiments there is provided use of the composition as described herein for preparation as a drug delivery platform.

According to some embodiments there is provided use of enzyme cross-linked albumin as a drug delivery platform.

According to some embodiments there is provided use of albumin and a cross-linking enzyme as a drug delivery platform.

According to some embodiments there is provided a method for cross-linking a globular protein, the method comprising adding to a solution of the globular protein a cross-linking enzyme and a structural modifier of the globular protein.

Prior to the herein described invention, enzyme-mediated polymerization of aqueous globular protein solutions has been demonstrated only at low protein concentrations, which require just mild denaturative treatment to denature globular proteins and facilitate enzyme crosslinking of the globular protein. These protein concentrations are too low to allow for the formation of a solid gel. At the higher protein concentrations that would be necessary to form a solid gel, higher levels of denaturative treatment have been used. The higher level of denaturative treatment itself, not enzymatic crosslinking, has resulted in aggregation or gelation of the protein.

As a result of the above-described technical limitations, the use of an enzymatically crosslinked globular protein gel has not previously been suggested for use in medical applications. Surprisingly, the inventors have determined that such an enzymatically crosslinked solid gel may be formed from globular proteins such that gelation occurs solely due to the function of the enzyme (without wishing to be limited by a single hypothesis) for the embodiments of the composition according to the present invention.

According to a preferred embodiment certain solutions of albumin which are denatured by incubation with a chaotropic agent only without heat treatment or disulfide bond reducing agent remain in a liquid state and become gel after addition of trans glutaminase. Preferably, the concentration of albumin for this type of solution is 5% w/w or above. More preferably, the concentration of albumin is 10% or above.

According to some embodiments of the present invention, the globular protein comprises soy protein, conalbumin, bovine serum albumin (BSA), human serum albumin, recombinant human albumin, hemoglobin, ovalbumin, α-chymotrypsinogen A, α-chymotrypsin, trypsin, trypsinogen, β-lactoglobulin, myoglobin, α-lactalbumin, lysozyme, ribonuclease A, or cytochrome c, or combinations thereof.

Optionally and preferably, the globular protein comprises bovine serum albumin.

Optionally and preferably, the globular protein comprises human derived serum albumin. According to some embodiments of the present invention, the cross-linking enzyme comprises transglutaminase.

According to preferred embodiments of the present invention, the cross-linking enzyme comprises calcium independent microbial trans glutaminase.

According to some embodiments, there is provided the use of any of the methods described herein in the preparation as a medical sealant.

According to some embodiments of the present invention, there is provided the use of cross-linked albumin as a medical sealant or glue.

According to some embodiments of the present invention, there is provided the use of albumin and a cross-linking enzyme as a medical sealant or glue.

According to some embodiments, there is provided the use of the medical sealant of the present invention in a medical application selected from the group consisting of reinforcement of surgical repair lines; provision of fluid-stasis; prevention of lymphorrhea; prevention of cerebro-spinal fluid (CSF) leakage; prevention of anastomotic dehiscence; and sealing of an attachment between a tissue and a material.

Optionally and preferably, the medical sealant is provided in a form selected from the group consisting of a gel, a spray, a strip, a patch, and a bandage.

According to some embodiments, there is provided the use of the methods of the present invention for preparation of a tissue engineering scaffold.

According to some embodiments of the present invention, there is provided the use of cross-linked albumin as a tissue engineering scaffold.

According to some embodiments of the present invention, there is provided the use of albumin and a cross-linking enzyme as a tissue engineering scaffold.

According to some embodiments, there is provided the use of the method of the present invention for preparation as a drug or polypeptide (or other biological) delivery platform. It should be noted that when reference is made to a "drug" any therapeutic agent is considered to be encompassed thereby, including but not limited to small molecules, large macromolecules such as macrolides, taxanes such as paclitaxel, polypeptides of any type whether linear or cyclic (such as cyclosporine, for example), peptides of any type, nucleotide-based agents and so forth.

According to some embodiments of the present invention, there is provided the use of cross-linked albumin as a drug or polypeptide delivery platform.

According to some embodiments of the present invention, there is provided the use of albumin and a cross-linking enzyme as a drug or polypeptide delivery platform.

According to a preferred embodiment, the globular protein solution or enzyme is preferably prepared for medical use.

According to some embodiments, the globular protein solution or enzyme are treated to reduce impurities, including but not limited to purities related to microorganisms. According to a preferred embodiment, the microbial colony forming unit (CFU) count of the globular protein solution or enzyme composition is reduced or eliminated through such treatment.

In a preferred embodiment, such treatment optionally comprises sterilizing the globular protein solution or enzyme composition through sterile filtration and/or radiation sterilization (gamma or ebeam).

According to a preferred embodiment, the endotoxin level of the globular protein solution or enzyme composition is reduced or eliminated. Optionally and preferably, the endotoxin level of the enzyme composition is reduced through cation exchange chromatography.

Unless defined otherwise, all technical and scientific terms used herein have the same meaning as is commonly understood by one of skill in the art to which this invention belongs. All patents, patent applications, and publications mentioned herein are incorporated herein by reference.

"Wound" as used herein refers to any damage to any tissue of a patient that results in the loss of blood from the circulatory system or the loss of any other bodily fluid from its physiological pathway. The tissue can be an internal tissue, such as an organ or blood vessel, or an external tissue, such as the skin. The loss of blood or bodily fluid can be internal, such as from a ruptured organ, or external, such as from a laceration. A wound can be in a soft tissue, such as an organ, or in hard tissue, such as bone. The damage may have been caused by any agent or source, including traumatic injury, infection or surgical intervention. The damage can be life-threatening or non-life-threatening.

" TG" refers to trans glutaminase of any type; "mTG" may also refer to microbial trans glutaminase and/or to any type of trans glutaminase, depending upon the context (in the specific experimental Examples below, the term refers to microbial transglutaminase).

"Gel" refers to a substantially dilute crosslinked system, which exhibits no flow when in the steady-state. It may also refer to the phase of a liquid achieved when a three-dimensional crosslinked network within the liquid develops, causing the elastic modulus of the composition to become greater than its viscous modulus.

"Denaturing agents" refers to substances that affect the structure of proteins. This group includes but is not limited to reducing agents, which disrupt the disulfide bonds in proteins, and chaotropic agents, which disrupt the hydrogen bonds in proteins.

As used herein, "about" means plus or minus approximately ten percent of the indicated value.

Other features and advantages of the invention will be apparent from the following detailed description, and from the claims.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in order to provide what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
Figures 1A and 1B show the results of a tensile strength test; and
Figures 2A and 2B show results of a Lap shear test.

### DESCRIPTION OF THE PREFERRED EMBODIMENTS

According to some embodiments of the present invention, there is provided a composition comprising a disulfide bond disrupting agent to prevent aggregation or gelation of a denatured globular protein in order to keep the denatured protein in solution. Denaturation may optionally be caused for example by a hydrogen bond disrupting method (heat and/or denaturing agent) at concentrations that would normally cause aggregation (precipitation or gelation) of a globular protein solution. This enables the formation of a solid protein gel through enzymatic crosslinking. As used herein the term "enzymatic crosslinking" refers to cross-linking which is functionally due to the effect of the enzyme, as opposed to the effect of denaturation itself. The resultant gel has many applications, including but not limited to applications in the medical and food industries as described herein.

Some non-limiting examples of these applications are given below. For example, the composition may optionally be used for the sealing of a vascular graft such as Dacron vascular graft in acute aortic dissection; as an adjunct to controlling air leaks on the lung parenchyma following resection; as a matrix for immobilization of cells or enzymes for in vivo, in vitro or ex vivo use as a bioreactor or biosensor; as a biomimetic scaffold for tissue engineering; as a platform for drug delivery; for preparation of albumin microspheres for controlled release of drugs; to construct a contact lens or other ophthalmic device; for artificial skin; as a wound dressing; as a surgical sealant along suture lines or about surgical staples, forming an anastomosis (the sutures or staples can be used, e.g., to join blood vessels, bowels, ureter, or bladder); for controlling or arresting organ bleeding; for coating the lumenal surface of a blood vessel, or other tissue cavity that has been damaged by trauma, surgical intervention, angioplasty or disease; for soft tissue augmentation or replacement applications including facial tissue augmentation, urinary incontinence; for plastic surgery reconstruction; as a spinal disc replacement; for lung volume reduction; to form albumin sponges and albumin-coated bandages for bleeding arrest and wound healing; to form electrospun albumin fibers; and to form albumin beads as packing material for HPLC columns and other chromatography applications.

Some non-limiting food industry applications include: encapsulation of food ingredients in albumin microspheres or nanospheres for: protection against oxidation, retention of volatile ingredients, taste masking, enhanced stability; gelation of food products, such as yoghurt-desserts; and stabilization of emulsions by acting as emulsifier to create emulsion gels.

Surprisingly, the disulfide bond disrupting agent, such as a reducing agent, does not need to be maintained at high concentrations in the solution in order to prevent gelation. For example, it may be removed through dialysis etc. This is completely novel and facilitates applications where a reducing agent might not be desirable (ie a food or medical application).

According to other embodiments of the present invention, there is provided an enzyme crosslinked solid globular protein gel for use in medical applications. All prior art is not appropriate for medical applications as a gel either because no gel was formed or because one or more non biocompatible materials were used.

According to still other embodiments of the present invention, there is provided an in situ enzymatic crosslinked globular protein gel, optionally and preferably cross-linked when in contact with the tissue of a subject, more preferably comprising a denaturing agent as described above. Optionally, this gel does not comprise a reducing agent and is not treated with heating.

According to some embodiments of the present invention, there is provided a method for cross-linking albumin for use as a sealant or glue for a biological system, for example to induce hemostasis and/or prevent leakage of any other fluid from a biological tube or tissue, such as lymph for example. The cross-linked albumin may optionally and preferably be applied as part of a bandage for example.

In other embodiments, the present invention provides a method of enzymatically cross-linking globular proteins, by altering the structure of the protein to improve the accessibility of the protein to the cross-linking enzyme.

According to some embodiments of the present invention, there is provided a method for cross-linking a globular protein, the method comprising adding to a solution of the globular protein a cross-linking enzyme and a structural modifier of the globular protein.

According to further embodiments of the present invention, there is provided a method for preparing a medical gel, the method comprising adding to a solution of a cross-linkable globular protein a cross-linking enzyme and a structural modifier of the globular protein.

According to some embodiments of the present invention, the structural modifier is a reducing agent.

The reducing agent is used to disrupt the disulfide bonds that are responsible for maintenance of the globular structure of globular proteins. By disrupting these disulfide bonds, and thereby modifying the structure of the globular protein, the cross-linker substrate sites on the protein are exposed.

Non-limiting examples of suitable reducing agents that can be used to disrupt the disulfide bonds of globular proteins include hexafluoroisopropanol (HFIP), dimethyl sulfoxide (DMSO), sodium dodecyl sulfate (SDS), glutathione, hydroquinone, 2-mercaptoethanol, 2-mercaptoethylamine, tris(2-carboxyethyl)phosphine hydrochloride (TCEP), cysteine, dithiothreitol (DTT), and N-ethylmaleimide.

Globular proteins which can be cross-linked in accordance with the principles of the present invention include, for example, soy protein, conalbumin, bovine serum albumin (BSA), human serum albumin, recombinant human albumin, hemoglobin, ovalbumin, α-chymotrypsinogen A, α-chymotrypsin, trypsin, trypsinogen, β-lactoglobulin, myoglobin, α-lactalbumin, lysozyme, ribonuclease A, and cytochrome c.

The cross-linking enzyme of the present invention is optionally and preferably transglutaminase (TG), which may optionally comprise any type of calcium dependent or independent transglutaminase (mTG), such as, for example, a microbial trans glutaminase.

Optionally, newly available commercial transglutaminase products containing 10% or more mTG may be used. Non-limiting examples of commercially available transglutaminase products of this sort include those produced by Ajinomoto Co. (Kawasaki, Japan) and Yiming Chemicals (China). A preferred example of such a product from this company is the Activa TG - Ingredients: mTG and maltodextrin; Activity: 810 - 1,350 U/g of Activa. Preferred products from Yiming include one product containing 10% mTG and 90% maltodextran and one product containing 10% mTG and 90% lactose, also of activity 810 - 1,350 U/g of product.

According to a preferred embodiment of the present invention, microbial transglutaminase (mTG) is used as the cross-linker and albumin as the protein. The mTG-substrates in the albumin that are exposed in accordance with this method are glutamine and lysine residue.

It should be noted that the specificity of microbial transglutaminase differs from that of tissue transglutaminase with regards to its ability to cross-link albumin. Some tissue transglutaminases are natively able to cross-link albumin to some extent. However, cross-linking of albumin using mTG requires treating the albumin with a reducing agent (de Jong GA, et al. J. Agric. Food Chem., 49 (7), 3389 -3393, 2001.).

Cross-linked globular proteins prepared in accordance with the method of the present invention have a wide range of applications for medical use, such as for a novel surgical sealant, tissue adhesive and hemostat. Other uses include tissue scaffolds and cell scaffolds.

Once the globular structure of a globular protein, such as albumin, has been disrupted and its mTG-substrates exposed, it provides an ideal protein for the preparation of adhesive compositions for use in soft tissue applications.

According to some embodiments there is provided a composition comprising a cross-linkable globular protein, a cross-linking enzyme and a structural modifier of the globular protein, for use in medical applications.

According to some embodiments, a gel prepared according to any of the methods of the present invention may be used in a medical application, such as for example, a medical sealant.

A medical sealant prepared according to the method of the present invention may be used, for example, in reinforcement of surgical repair lines (including staple lines or suture lines); for providing fluid-stasis, such as hemostasis or lymphostasis; for preventing lymphorrhea; for prevention of cerebro-spinal fluid (CSF) leakage; for preventing anastomotic dehiscence; or for sealing an attachment between a tissue and a material, including another tissue, an implant, a prosthesis, or a skin graft.

The medical sealant may be provided, for example, in the form of a gel, a foam, a spray, a strip, a patch, or a bandage.

According to some embodiments of the present invention, the composition is provided in a bandage, which is preferably adapted for use as a hemostatic bandage. The herein described compositions may additionally have one or more uses including but not limited to tissue adhesives (particularly biomimetic tissue adhesives), tissue culture scaffolds, tissue sealants, hemostatic compositions, drug delivery platforms, surgical aids, or the like, as well as other non-medical uses, including but not limited to edible products, cosmetics and the like, such as, for example, in purification of enzymes for use in food products.

### EXAMPLES

Reference is now made to the following examples, which together with the above description, illustrate some embodiments of the invention in a non limiting fashion. Unless otherwise indicated, all of the below Examples relate to one or more of the below list of Materials.

### Materials:

Bovine Albumin Fraction V 96%-99% [Biological Industries, Israel, Lot#700118], Sodium Acetate (x3H20 C.P. lot #104013), Dulbecco's Phosphate Buffered Saline without Calcium and Magnesium [Biological Industries, Israel], microbial Transglutaminase ACTIVA-TG 10% enzyme powder in maltodexterin [Ajinomoto, Japan], urea (Sigma), TCEP (Sigma), 2-mercaptoethanol (Aldrich chemicals), collagen strips (Nitta Casings) and NaCl (Frutarom, Israel).

Without wishing to be limited in any way, when TCEP and urea are used together, the following characteristics preferably apply: the ratio of urea to albumin is optionally 10-150 mmols urea /gram albumin, preferably 25-120 mmols urea/ gram albumin. The ratio of TCEP to albumin is optionally 0.1-1.5 mmols TCEP/ gram albumin, preferably 0.25-1.25mmols TCEP/gram albumin. The Molar Ratio of urea to TCEP is optionally 10 to 1000 and preferably is 20-500.

### Example 1: Effect of TCEP on heat and urea induced gelation of BSA

Example 1 shows that the aggregation of albumin as a result of heat treatment and the presence of urea can be reversed by addition of a reducing agent such as TCEP. It also shows, without wishing to be limited by a single hypothesis, that urea has a major role in maintaining the resulting solution in a liquid state.

Furthermore, this Example describes removal of TCEP from the reaction mixture by dialysis. Removal of the disulfide reducing agent in other types of solutions is sometimes accompanied by air oxidation of the thiols back to the disulfides. Surprisingly, after the removal of TCEP by extensive dialysis the dialyzate remained in a liquid form.

2 gram BSA were mixed with 38 gram 5.6M urea to yield 5% w/w BSA solution (Solution A). The solution was heated at 70°C. After 15 minutes the solution started to get cloudy and contained white aggregates. After 25 minutes at 70°C 2ml of 1M TCEP were added to yield Solution B (50mM final concentration) and as a result the solution clarified immediately. This shows that TCEP is required to open S-S bonds which form as a result of heating in the presence of urea. 5ml of Solution B were dialyzed against 500 ml 4M urea for 1 hour with 1 buffer change followed by 16 hour further dialysis. At the end of this dialysis step the dialysate was in a liquid form.

Next, dialysis was performed against 500ml 2M urea for 2 hours At the end of this dialysis step the dialysate was in a liquid form. Next, dialysis was performed against 1 liter of water for 2 hours, such that the urea was effectively dialyzed out of the protein solution. The dialyzate from this stage turned to a soft and flowing gel (data not shown). This shows that the urea is required to keep denatured BSA in liquid form and that TCEP can be removed from the mixture as long as there is enough urea to keep the denatured BSA in a liquid form

### Example 2: Effect of TCEP concentration on physical state of BSA

Example 2 shows that inclusion of TCEP at a concentration greater than at least 12mM can prevent heat- and urea- induced aggregation or gelation of a 5% BSA solution, thereby demonstrating the overall efficacy of TCEP as a gelation controlling agent.

5% w/w BSA containing 5.6M urea was mixed with various concentrations of TCEP followed by heating at 70°C for 10 minutes. The physical state of the BSA+urea+TCEP solution after the heating step is described in the table below.

**Table 1 - TCEP effect**

| **TCEP concentration (mM)** | **Physical state of the BSA+urea+TCEP solution after heating at 70°C for 10 minutes** |
|---|---|
| 50 | Clear liquid |
| 25 | Clear liquid |
| 20 | Clear liquid |
| 15 | Clear liquid |
| 12.5 | Clear liquid |
| 10 | Viscous clear liquid |
| 7.5 | Very Viscous clear liquid - almost a gel |
| 5 | Transparent gel |
| 2.5 | Opaque gel |
| 0 | White solid |

### Example 3: Effect of heating and various combination of BSA, urea and TCEP concentration on the physical state of BSA

Example 3 shows that the physical state of an albumin solution that has been heat treated for 10 minutes at 70°C shows a direct concentration dependence on urea and TCEP (greater amounts of urea and TCEP result in a more liquid state) and on the concentration of albumin in an inverse correlation (more albumin results in aggregation/gelation). Without wishing to be limited by a single hypothesis, it may be the molar ratio of urea and TCEP to albumin that determines the physical state.

BSA solutions containing urea were heated for 10 minutes at 70°C in the presence of TCEP and the physical state of the solution was recorded.

**Table 2 - TCEP/Urea vs Protein Concentration**

| **BSA %** | **Urea conc. (M)** | **TCEP conc. (mM)** | **Physical state after 10 min at 70°C** |
|---|---|---|---|
| 16 | 4.5 | 50 | Clear gel |
| 16 | 4.5 | 10 | Clear gel |
| 16 | 4.5 | 2 | White solid |
| 16 | 2.25 | 50 | Clear gel |
| 16 | 2.25 | 10 | White solid |
| 16 | 2.25 | 2 | White solid |
| 16 | 1.125 | 50 | Clear gel |
| 16 | 1.125 | 10 | White solid |
| 16 | 1.125 | 2 | White solid |
| 8 | 4.5 | 50 | Clear liquid |
| 8 | 4.5 | 10 | Clear gel |
| 8 | 4.5 | 2 | White solid |
| 8 | 2.25 | 50 | Clear gel |
| 8 | 2.25 | 10 | Clear gel |
| 8 | 2.25 | 2 | White solid |
| 8 | 1.125 | 50 | Clear gel |
| 8 | 1.125 | 10 | White solid |
| 8 | 1.125 | 2 | White solid |
| | | | |
| 4 | 4.5 | 50 | Clear liquid |
| 4 | 4.5 | 10 | Clear liquid |
| 4 | 4.5 | 2 | Clear gel |
| 4 | 2.25 | 50 | Clear liquid |
| 4 | 2.25 | 10 | Clear semi-gel |
| 4 | 2.25 | 2 | White solid |
| 4 | 1.125 | 50 | Clear liquid |
| 4 | 1.125 | 10 | Clear semi-gel |

### Example 4: Effect of TCEP concentration on mTG dependent gelation of BSA:

This Example shows that disulfide bond reducing agents have an inhibitory effect on microbial trans glutaminase.

500 ul 4% w/w solution containing 4.5M urea was heated at 70°C for 3 minutes until precipitation occurred. Next, 25ul of TCEP at various concentrations was added and the effect on the precipitated material was recorded. Next, 100 ul of 0.75% w/w mTG (7.5% w/w ACTIVA-TG 10%) was added to reactions that were clarified by the addition of TCEP and the reactions were incubated at 37°C. The results show that TCEP is inhibitory to mTG-induced gelation in a dose dependent manner.

**Table 3 - TCEP inhibition of gelation**

| **TCEP concentration (mM)** | **Physical state after adding TCEP** | **After incubation with 100ul mTG** |
|---|---|---|
| 50 | Clarified immediately | Did not gel |
| 25 | Clarified immediately | Did not gel |
| 10 | Clarified after 5 minutes | Soft gel after 75 min |
| 5 | Hardly clarified | Not determined |
| 0 | Did not clarify | Not determined |

### Example 5: Both chaotropic agents and reducing agents are required for mTG dependent crosslinking of albumin

4 gram BSA were mixed with 16 gram water, resulting in a 20% w/w BSA solution (solution A). 8 grams of urea was added to 18 ml of Solution A to yield Solution B. Final volume of solution B was 23 ml. The urea concentration in Solution B was 5.8M and the BSA was 13.8% w/w.

1.9 ml of 5.6M urea solution was added to 5 ml of Solution B to yield a 10% w/w BSA + 5.75 M urea (Solution C).

0.275 ml of 1M TCEP solution was added to 5.5 ml Solution C to yield Solution D. The final concentration of TCEP in Solution D was 47.6mM. Solution D was heated at 70°C for 15 minutes with constant agitation. The solution remained a clear liquid after the heating step.

The heated Solution D was dialyzed against 500 ml of 5.6M urea at room temperature for 2.33 hours. The buffer was changed to a new 5.6M urea and dialysis continued for 2 more hours. The dialyzate was heated at 70°C for 15 minutes but remained a clear liquid after the heating step.

The dialyzate was incubated at 37°C in 3 different reactions:
Reaction #1: 500 ul dialyzate+ 125 ul 1.4% w/w mTG in water (14% w/w ACTIVA-TG 10% ): gel was formed after <15 min.
Reaction #2: 500 ul dialyzate+ 250 ul 1.4% w/w mTG in water (14% w/w ACTIVA-TG 10% ): gel was formed after <15 min.
Reaction #3 (control) : 500 ul dialyzate+ 250 ul water: No gel was formed , the solution is a viscous liquid.

Solution C (without TCEP, without dialysis) was incubated in 2 different reactions:
Reaction #4: 500 ul Solution C+ 125 ul water
Reaction #5: 500 ul Solution C+ 125 ul 1.4% w/w mTG in water (14% w/w ACTIVA-TG 10%)

The resultant solutions after both Reactions #4 and #5 were liquid after 5.5 hours at 37°C followed by 36 hours at room temperature.

The above experiment shows that in addition to urea, mTG dependent gelation is dependent also on treatment of BSA with the reducing agent TCEP and/or heat treatment and that urea alone at this BSA concentration (10% w/w) is not enough to render albumin cross-linkable by mTG.

### Examples 6 and 7 - effect of manipulating thiol bond formation

Examples 6 and 7 relate to the effect of manipulating thiol bond formation in terms of increasing or decreasing gelation. In contrast to TCEP which protects albumin from heat-induced aggregation in the presence of urea, both DTT and 2-mercaptoethanol trigger heat-induced protein aggregation in the presence of urea at temperatures where urea alone or reducing agent alone do not cause aggregation, e.g. 50°C.

The aggregation, precipitation and gelation that occur as a result of incubation with disulfide bond reducing agents such as DTT and 2-mercaptoethanol may be prevented through the addition of salt (Lee et al, Agricultural and Biological Chemistry ,Vol.55 , No.8(1991) pp.2057-2062). The salt may prevent intermolecular interaction of disulfide-reduced protein. The results below relate to mTG-dependent gelation of a solution of BSA that has been denatured with a combination of heating, urea and 2-ME (2-mercaptoethanol), and optionally salt.

### Example 6: Prevention of thiol-induced gelation of BSA by salt

A solution of 10% w/w BSA and 5.6M urea was incubated with 2-mercaptoethanol and NaCl in various combinations at different temperatures. The physical state of the solution after heating was recorded. Tables 4 and 5 show that there is a clear concentration dependence of gelation on the concentration of salt; however, treatment with sufficient heating could overcome the presence of salt.

**Table 4 - Effect of salt on gelation**

| **Reaction #** | **2-mercaptoethanol** | **NaCl** | **50°C, 10 minutes** | **60°C, 10 minutes** |
|---|---|---|---|---|
| 1 | - | - | Clear liquid | White solid |
| 2 | - | 400 mM | Clear liquid | Clear solid gel |
| 3 | 50 mM | - | White solid | White solid |
| 4 | 50 mM | 400 mM | Clear viscous liquid | White solid |

**Table 5 - effect of different salt concentrations on gelation**

| **Reaction #** | **2-mercaptoethanol** | **NaCl** | **50°C, 25 minutes** |
|---|---|---|---|
| 1 | 2 mM | - | Opaque gel |
| 2 | 2 mM | 62.5 mM | Clear gel |
| 3 | 2 mM | 125mM | Clear gel |
| 4 | 2 mM | 250 mM | Clear liquid |
| 5 | 2 mM | 500 mM | Clear liquid |

### Example 7: mTG- and urea-dependent gelation using 2-mercaptoethanol as reducing agent

### Reactions with urea

Solution 1: 8.33% BSA w/w, 4.67M urea, 500mM NaCl, 1.67mM 2-mercaptoethanol
Solution 2: 8.33% BSA w/w, 4.67M urea, 250mM NaCl, 1.67mM 2-mercaptoethanol
The solutions were heated at 50°C for 25 minutes. They were both clear liquid.
Reaction A: To 500 ul of solution 1, 125 ul water was added.
Reaction B: To 500 ul of solution 1, 125 ul 1.4% w/w mTG (14% w/w ACTIVA-TG 10%) was added.
Reaction C: To 500 ul of solution 2, 125 ul water was added.
Reaction D: To 500 ul of solution 2, 125 ul 1.4% w/w mTG (14% w/w ACTIVA-TG 10%) was added.
Reactions A through D were incubated at 40°C for 3 hours.
Reactions B and D were both turned to gels. The control reactions A and C were liquid.

Solution 3: 8.33% BSA w/w, 4.67M urea, 8.33mM 2-mercaptoethanol, 500 mM NaCl
Solution 4: 8.33% BSA w/w, 4.67M urea, , 8.33 mM 2-mercaptoethanol
The solutions were heated at 50°C for 15 minutes. Solution 4 turned to opaque gel. Solution 3 remained a clear liquid.
Reaction E: To 500 ul of solution 3, 125 ul water was added.
Reaction F: To 500 ul of solution 3, 125 ul 1.4% w/w mTG (14% w/w ACTIVA-TG 10%) was added
Reactions E and F were incubated at 40°C. After 3 hours both reactions were a viscous liquid. The reactions were then incubated at room temperature for 62 hours and both turned to gel.

### Reaction without urea:

Solution 5: 8.33% BSA w/w, 1.67mM 2-mercaptoethanol, 500mM NaCl
Solution 6: 8.33% BSA w/w, 1.67mM 2-mercaptoethanol, 250mM NaCl
Solution 7: 8.33% BSA w/w, 1.67mM 2-mercaptoethanol
Solution 8: 8.33% BSA w/w

The solutions were heated at 50°C for 32 minutes and then at 60°C for 38 minutes. They were all clear liquid.

To 500 ul of solutions 5 to 8, 125 ul 1.4% w/w mTG (14% w/w ACTIVA-TG 10%) was added. The reactions were incubated at 40°C for but did not gel after 20 hours incubation.

Solutions 5-8 were also heated at 70°C for 30 minutes. Reactions 7 and 8 precipitated, but reactions 5 and 6 remained each a clear liquid.

To 500 ul of solutions 5 and 6 that were heated at 70°C, 125 ul 1.4% w/w mTG (14% w/w ACTIVA-TG 10%) was added. The reactions were incubated at 40°C for but did not gel after 20 hours incubation.

These results demonstrate that mTG-induced gelation of BSA in the presence of 2-mercaptoethanol can be achieved by inclusion of urea. Without urea higher concentrations of 2-mercaptoethanol and higher temperatures are required in order to denature the protein, but the mTG enzyme is sensitive to high concentrations of 2-mercaptoethanol. Also, high concentration of 2-mercaptoethanol may result in mTG-independent gelation as demonstrated by reactions E and F.

### Example 8: mTG-dependent gelation of BSA in the presence of urea but without heat treatment and reducing agent

This Example demonstrates that certain solutions of albumin which are denatured by incubation with a chaotropic agent only without heat treatment or disulfide bond reducing agent remain in a liquid state and become gel after addition of trans glutaminase. Preferably, the concentration of albumin for this type of solution is 5% w/w or above. More preferably, the concentration of albumin is 10% or above.

12.5 gr BSA were added to 37.5 gr water to produce a 25% w/w solution. The solution volume was 42 ml.

17.1 gr urea were added to the 25% BSA solution. The final volume was 54ml, the BSA concentration 18.6% w/w and the urea concentration 5.3M.

125 ul of mTG 1.4% w/w (14% w/w ACTIVA-TG 10%) were added to 500 ul BSA+urea solution and the mixture incubated at 40°C. As a control reaction, water was used instead of mTG.

After 20 minutes the reaction with mTG was very viscous and at 25 minutes it became a soft gel.

The control reaction with water was a non-viscous liquid after 8 hours incubation.

### Example 9: Mechanical testing of BSA gels similar to those formed in Example 8

Example 9 shows the mechanical properties of gels made from solutions of albumin and urea as described in Example 8. Gels prepared this way show both cohesive and adhesive strength and are therefore suitable for medical applications, for example.

1.5ml of 1.4% mTG in water (14% w/w ACTIVA-TG 10%) was mixed with 6ml of 18.6% w/w BSA and 5.3M urea solution. The resulting mixture was applied to dog bone shaped molds, with 2 mL in each mold. The effective testing cross-sectional area of the gels formed in these molds was 12 mm by 1.7 mm. Molds containing gels were incubated at 37 °C. After 40 minutes the mixture turned to a soft gel. The gels were further incubated for 40 more minutes, then the molds were covered in saline and the formed gels were extracted from the molds.

For the testing of each gel, the tabs on either end of the dogbone shaped gel were clamped into a Model 3343 Single Column Materials Testing System (Instron™; Norwood, MA). The top tab was then pulled upwards at a rate of 0.5 mm/s, resulting in the creation of tensile force on the gel dogbone. Tension of sample was continued until failure was observed. Bluehill 2 Materials Testing Software (Instron™; Norwood, MA) was used to analyze results and calculate material properties including elastic modulus, peak stress, and strain to break.

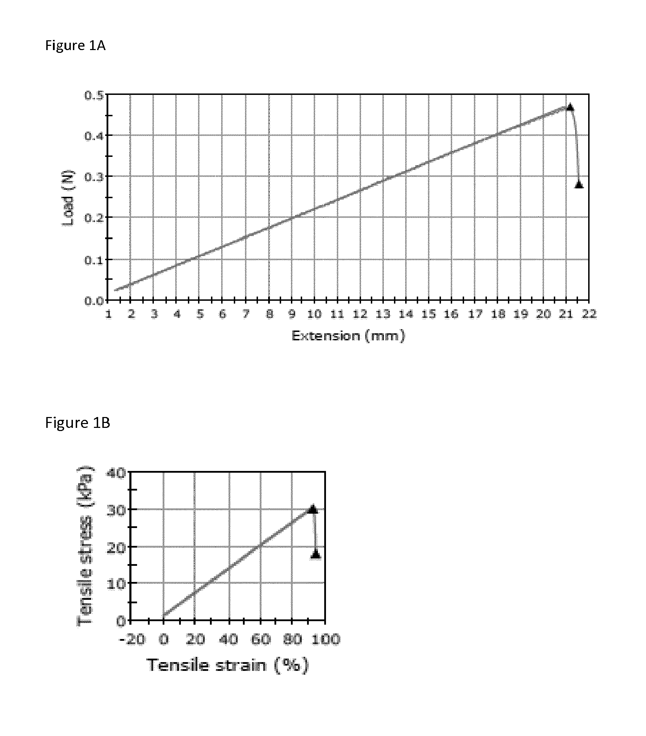

The results are shown in Figures 1A and 1B, and in Table 6 below.

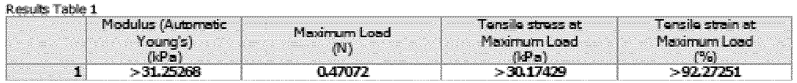

As shown, Figure 1A demonstrates that the tensile strength permitted a maximum load of 0.47072, while Figure 1B shows that the tensile stress at maximum load was greater than 30 kPa, and that the tensile strain at the maximum load was over 90%. In combination, these results show that the resultant gel was both very strong and very flexible, and was capable of absorbing high levels of strain and stress without breaking or otherwise failing.

Next, 0.25 ml of 14% mTG in water (14% w/w ACTIVA-TG 10% )was mixed with 1ml of 18.6% w/w BSA and 5.3M urea solution.

0.3ml of the mixture were applied on a 2cm x 2cm area between two 5cm x 2cm strips of collagen (Edible Collagen Casing, Nitta Casings Inc.) that were presoaked with saline. The collagen strips were incubated at 37°C for 1 hour, allowing the albumin to gel. The adhesive strength was measured by the Lap Shear test as follows. For the testing of each gel, the edges of the collagen strips were clamped into a Model 3343 Single Column Materials Testing System (Instron™; Norwood, MA). The top strip was then pulled upwards at a rate of 0.5 mm/s, resulting in the creation of tensile force on the gel-containing overlapping section of the collagen strips. Tension of sample was continued until failure was observed. Bluehill 2 Materials Testing Software (Instron™; Norwood, MA) was used to analyze

results and calculate material properties including elastic modulus, peak stress, and strain to break.

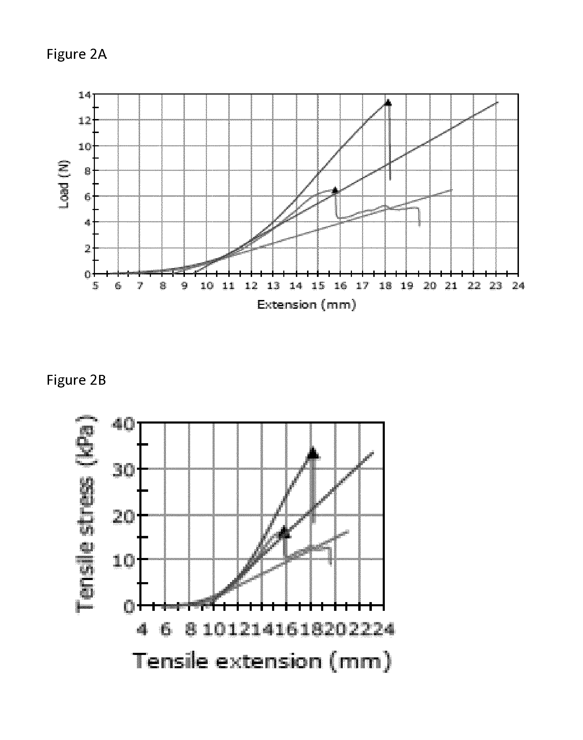

The results are shown in Figures 2A and 2B, and also Table 7 below.

**Table 7:**

| Test # | Tensile stress at maximum load (kPa) | Tensile strain at maximum load (%) |
|---|---|---|
| 1 | 16.38 | 78.7 |
| 2 | 33.47 | 87.3 |

The results show that the resultant gel was both very strong and very flexible, and was capable of absorbing high levels of strain and stress without breaking or otherwise failing.

### Example 10: Cross-linking of albumin

This Example shows that unmodified globular proteins, such as bovine albumin do not undergo cross-linking in the presence of microbial transglutaminase (mTG), either in sodium acetate or Dulbecco's phosphate buffered saline buffers, thereby demonstrating that this inability to undergo cross-linking is not affected by the choice of buffer.

0.1M Sodium Acetate solution at pH 6.0 was prepared. 0.075% mTG solution in sodium acetate buffer was prepared.
25% (w/w) albumin solutions were prepared into the following solutions:
Solution A- bovine albumin in 0.1M sodium acetate solution.
Solution B- bovine albumin in 0.1M Phosphate Buffered Saline solution.

### Results:

Table 8 below shows cross-linking times of albumin using microbial transglutaminase. Cross-linking is defined as the time in which a massive gelatinous mass is formed.

As illustrated in the table, 25% w/w albumin in 0.1M Phosphate Buffered Saline buffer or in 0.1M sodium acetate buffer does not form cross-links in the presence of mTG. No cross-linking occurred in 2 hours after the albumin solution. Solutions were examined the following morning and still no cross-linking had occurred.

**Table 1**

| Solution | Test # | Cross-linking Time (min) | Description of Cross-Linked Gel |
|---|---|---|---|
| A | 1 | Did not cross-link | Solution did not cross-link and remained liquid for the duration of the test (2 hours) |
| A | 2 | Did not cross-link | Solution did not cross-link and remained liquid for the duration of the test (2 hours) |
| A | 3 | Did not cross-link | Solution did not cross-link and remained liquid for the duration of the test (2 hours) |
| B | 1 | Did not cross-link | Solution did not cross-link and remained liquid for the duration of the test (2 hours) |
| B | 3 | Did not cross-link | Solution did not cross-link and remained liquid for the duration of the test (2 hours) |
| B | 3 | Did not cross-link | Solution did not cross-link and remained liquid for the duration of the test (2 hours) |

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention.

## Claims

1. A gel composition comprising albumin and an enzymatic cross-linker for use as a medical sealant, wherein said albumin has undergone reduction and denaturation.

2. The composition of claim 1, wherein said albumin has been reduced using one or more reducing agents selected from the group consisting of hexafluoroisopropanol (HFIP), dimethyl sulfoxide (DMSO), sodium dodecyl sulfate (SDS), hydroquinone, 2-mercaptoethylamine, and N-ethylmaleimide.

3. The composition of claim 1, wherein said albumin has been denaturated using one or more denaturing agents selected from the group consisting of a chaotropic agent, a disulfide bond reducing agent or some combination of these.

4. The composition of claim 3, wherein said chaotropic agent is selected from the group consisting of urea, guanidinium chloride, and lithium perchlorate, and/or wherein said disulfide bond reducing agent comprises one or more of a thiol containing reducing agent, including but not limited to 2-mercaptoethanol, dithiothreitol (DTT), cystein, glutathione, a phosphine-containing agent or a combination thereof.

5. The composition of claim 4, wherein the ratio of urea to albumin is in the range of 10-150 mmols urea /gram albumin; and/or wherein the ratio is in the range of 25-120 mmols urea/ gram albumin.

6. The composition of claim 4, wherein said phosphine containing agent comprises tris(2-carboxyethyl) phosphine (TCEP); and/or wherein said reducing agent is selected from the group consisting of hexafluoroisopropanol (HFIP), dimethyl sulfoxide (DMSO), sodium dodecyl sulfate (SDS), hydroquinone, 2-mercaptoethylamine, and N-ethylmaleimide.

7. The composition of claim 6, wherein the ratio of TCEP to albumin is 0.1-1.5 mmols TCEP/ gram albumin; or optionally wherein the ratio is in the range of 0.25-1.25 mmols TCEP/gram albumin; and/or wherein the ratio is in the range of 10-30 micromoles 2-mercaptoethanol / gram albumin.

8. The composition of claim 1, wherein said enzymatic cross-linker comprises trans glutaminase.

9. The composition of claim 8, wherein said transglutaminase is calcium independent.

10. The composition of claim 8, wherein said transglutaminase is microbial trans glutaminase.

11. The composition of claim 8, wherein a protein content of said trans glutaminase is present in an amount from about 0.0001% to about 2 % w/w of the composition.

12. The composition of claim 8, wherein the concentration of transglutaminase is in the range of from about 5 to about 100 enzyme units (U/mL) of total composition.

13. The composition of claim 1, wherein said albumin is present in an amount from about 2% to about 25 % w/w of the gel composition.

14. The composition of claim 1, wherein said medical sealant is used in a medical application selected from the group consisting of reinforcement of surgical repair lines; provision of fluid-stasis; prevention of lymphorrhea; prevention of cerebro-spinal fluid (CSF) leakage; prevention of anastomotic dehiscence; and sealing of an attachment between a tissue and a material.

15. The composition of claim 1, wherein said medical sealant is provided in a form selected from the group consisting of a gel, a spray, a strip, a patch, and a bandage.
